# EUROPÄISCHE PATENTSCHRIFT

(11) **EP 3 762 718 B1**
(45) Veröffentlichungstag und Bekanntmachung des Hinweises auf die Patenterteilung: **21.10.2026**
(21) Anmeldenummer: 19710655.2
(22) Anmeldetag: 08.03.2019
(51) Int. Cl.: G01N 33/08

(54) **VERFAHREN ZUR IN-OVO FERTILISATIONS- UND GESCHLECHTSBESTIMMUNG AM GESCHLOSSENEN EI**
METHOD FOR IN-OVO FERTILISATION DETERMINATION AND GENDER DETERMINATION ON A CLOSED EGG
PROCÉDÉ DE FÉCONDATION IN OVO ET DE SEXAGE DANS L'OEUF FERMÉ

(30) Priorität: 09.03.2018 DE 102018105512
(43) Veröffentlichungstag der Anmeldung: 13.01.2021
(73) Patentinhaber: Technische Universität Dresden, 01069 Dresden (DE)
(72) Erfinder: PREUSSE, Grit, 01445 Radebeul (DE); STEINER, Gerald, 08340 Schwarzenberg (DE); GALLI, Roberta, 01309 Dresden (DE); KOCH, Edmund, 01307 Dresden (DE); SCHNABEL, Christian, 01099 Dresden (DE)
(74) Vertreter: Rauschenbach, Marion
(86) Internationale Anmeldenummer: PCT/EP2019/055904
(87) Internationale Veröffentlichungsnummer: WO 2019/170887

(56) Entgegenhaltungen:
- WO-A1-2018/023105
- DE-A1- 102012 023 947
- DE-A1- 102014 010 150
- DE-B3- 102010 006 161
- DE-B3- 102012 016 911
- US-A1- 2016 239 953
- ROBERTA GALLI ET AL: "In Ovo Sexing of Domestic Chicken Eggs by Raman Spectroscopy", ANALYTICAL CHEMISTRY, vol. 88, no. 17, 6 September 2016 (2016-09-06), US, pages 8657 - 8663, XP055378439, ISSN: 0003-2700, DOI: 10.1021/acs.analchem.6b01868
- GALLI ROBERTA ET AL: "In ovo sexing of chicken eggs by fluorescence spectroscopy", ANALYTICAL AND BIOANALYTICAL CHEMISTRY, SPRINGER, DE, vol. 409, no. 5, 14 December 2016 (2016-12-14), pages 1185 - 1194, XP036142794, ISSN: 1618-2642, [retrieved on 20161214], DOI: 10.1007/S00216-016-0116-6
- GERALD STEINER ET AL: "Gender determination of fertilized unincubated chicken eggs by infrared spectroscopic imaging", ANALYTICAL AND BIOANALYTICAL CHEMISTRY, SPRINGER, BERLIN, DE, vol. 400, no. 9, 9 April 2011 (2011-04-09), pages 2775 - 2782, XP019909218, ISSN: 1618-2650, DOI: 10.1007/S00216-011-4941-3

## Beschreibung

Die Erfindung bezieht sich auf die Gebiete der Verfahrenstechnik und der Landwirtschaft und betrifft ein Verfahren zur in-ovo Fertilisations- und Geschlechtsbestimmung am geschlossenen Ei. Das Verfahren kann zur in-ovo Fertilisations- und Geschlechtsbestimmung beispielsweise bei Vogeleiern Verwendung finden.

Eine hohe negative Korrelation zwischen Legeleistung und Mastleistung verhindert die gleichzeitige Nutzung des Haushuhnes als Eierproduzent und Fleischlieferant. So entstanden in den letzten Jahrzehnten durch Züchtung spezialisierte Linien von Wirtschaftshühnern, die entweder eine ökonomische Produktion von qualitativ hochwertigen Eiern ermöglichen (Hennen) oder aber sich für eine effiziente Fleischproduktion eignen (Hähne und Hennen). Während die Hennen der Mastlinien ebenfalls gemästet werden, können Hähne aus Legelinien gegenwärtig nur in sehr geringem Umfang wirtschaftlich aufgezogen und vermarktet werden. Von dieser Problematik ist nicht nur die konventionelle Eierproduktion betroffen, sondern auch die nach ökologischen Richtlinien wirtschaftende Legehennenhaltung. Das bisher gängige Verfahren, die männlichen Nachkommen von Legehennenlinien bereits unmittelbar nach dem Schlupf zu töten, steht im Widerspruch zu den gesetzlichen Bestimmungen und Zielen des Tierschutzes und stößt damit sowohl im In- als auch im Ausland auf ethische und rechtliche Bedenken. Deshalb gibt es zunehmend Forschungsaktivitäten hinsichtlich Alternativen zur Tötung männlicher Eintagsküken bei der Produktion von Eiern. Bisher wurden invasive und nichtinvasive Methoden zur Geschlechtsbestimmung von befruchteten Hühnereiern in verschiedenen Bebrütungszeiträumen beschrieben.

Aus der DE 10 2014 010 150 A1 ist ein Verfahren und eine Vorrichtung zur Ramanspektroskopischen in-ovo Geschlechtsbestimmung von befruchteten und bebrüteten Vogeleiern bekannt, bei dem ein Loch in die Kalkschale durch einen Laser oder mechanische Perforation eingebracht wird und die Ramanstreustrahlung des bestrahlten Blutes erfasst und mittels Auswerteeinheit ausgewertet wird.

Ein kontaktloses spektroskopisches Verfahren und eine Vorrichtung zur optischen in-ovo Geschlechtsbestimmung von befruchteten und bebrüteten Vogeleiern wird in der DE 10 2016 004 051 A1 beschrieben. Ebenfalls wird ein Loch in die Kalkschale eingebracht und die Geschlechtsbestimmung erfolgt hierbei durch Auswertung der Ramanstreustrahlung am Bruttag 4, was einem frühen Embryonalstadium vor Einsetzen des Schmerzempfindens entspricht.

Nachteilig ist bei diesem Verfahren, das vor der spektroskopischen Messung in die Kalkschale ein Loch eingebracht werden muss, um die Geschlechtsbestimmung durchführen zu können.

Der Nachteil der invasiven Verfahren besteht in einem erhöhten Infektions-und Verletzungsrisiko sowie in einem erhöhten technischen Aufwand für die Eiöffnung und den Eiverschluss.

In Galli et.al. [Anal Bioanal Chem. 2017 Feb; 409(5):1185-1194] wird die Geschlechtsspezifik der Fluoreszenzspektren von embryonalem Hühnerblut nach Einbringen einer Öffnung in die Eischale beschrieben, die sich in der spektralen Form und den Intensitätswerten zeigt. Entsprechend der Theorie verändert sich die Struktur eines Moleküls durch einen elektronischen Übergang in der Regel kaum. Deshalb besitzt der angeregte energetische Zustand eine dem Grundzustand ähnliche Molekülstruktur. Die Feinstruktur der Energieniveaus von Grund- und angeregten Zustand ist in der Regel sehr ähnlich. Daraus leitet sich die oft beobachtete Spiegelsymmetrie zwischen dem Fluoreszenzemissionsspektrum und dem Absorptionsspektrum im Bereich der S1/S0 -Übergänge ab.

Um eine gesunde Embryonalentwicklung zu gewährleisten und eine ökonomisch sinnvolle Lösung technisch realisieren zu können, wird seit Jahren die Geschlechtsbestimmung am geschlossenen Vogelei zu einem möglichst frühzeitigen Entwicklungszustand angestrebt, auch im nicht angebrüteten Ei.

Von Imholt, D. ("Morphometrische Studien an Eiern von Hybrid- und Rassehühnern mit Versuchen zur Detektion einer Beziehung zwischen der Form von Eiern und dem Geschlecht der darin befindlichen Küken", Inauguraldissertation, VVB Laufersweiler Verlag, 2010) ist bekannt, dass kein Zusammenhang zwischen der Form und Größe des Eies zum Geschlecht des Embryos gefunden werden konnte.

Aus der WO 2014 086 335 A1 sind ein Verfahren und eine Anordnung zur nichtinvasiven, zerstörungsfreien Identifikation molekülspezifischer und/oder biologischer Eigenschaften einer inneren Struktur eines biologischen Untersuchungsobjektes durch eine optisch undurchlässige Barriere hindurch bekannt, bei dem das Untersuchungsobjekt, wie beispielsweise ein Ei, nicht verändert, also die Eischale nicht geöffnet wird. Dabei wird das Ei unter einem Array von Pulssendern und Empfängern positioniert, wobei die von den Pulssendern ausgesendeten elektromagnetischen Pulse im Spektralbereich 0,01 bis 1 THz liegen. Die vom Untersuchungsobjekt ausgehende Strahlung wird von den Empfängern aufgenommen und im Computer ausgewertet. Die Auswertung erfolgt durch den rechnergestützten Vergleich der erfassten THz-Absorptionsspektren mit Referenzspektren. Zur Extraktion des relevanten Einzelspektrums des genetischen Materials der Keimscheibe werden dabei Spektren der Einzelkomponenten des Vogeleis berechnet und vom gemessenen integralen Spektrum subtrahiert. Auf Basis adaptiver Software-Algorithmen wird das Modell innerhalb einer Charge verfeinert. Zum Ende der Messung einer Charge wird das Datenaufkommen ausgewertet und mit statistischen Methoden unter der Voraussetzung zu erwartender geschlechtsspezifischer Merkmale analysiert.

Nachteilig dabei ist, dass der Anteil der Absorption der Keimscheibe mit den DNAhaltigen Zellen im Vergleich zur Summe der Absorptionen aus den anderen Eikomponenten (Eigelb, Eiweiß, Eischale) prozentual jedoch sehr gering ist.

Bei Anwendung der als "genetic engineering" bekannten Verfahren und dem damit verbundenen Einsatz transgener Tiere ist eine Geschlechtsbestimmung im befruchteten, nicht angebrüteten Hühnerei möglich.

Aus der WO 2017 094 015 A1 ist ein Verfahren zur Geschlechtsbestimmung in unbebrüteten Vogeleiern bekannt, bei dem mindestens ein Reportergen in mindestens eines der Geschlechtschromosomen W oder Z integriert wird. Das optische Signal dieses Reportergens, beispielsweise initiiert durch Biolumineszenz, kann durch die Eischale detektiert werden.

Die hyperspektrale Bildgebung (hyperspectral imaging, kurz: HSI) stellt eine weitere Methode dar, die angewendet wurde, um an geschlossenen Hühnereiern eine Bestimmung der Befruchtungsrate und des Geschlechtes zu einem möglichst frühzeitigen Bebrütungszustand durchführen zu können. Bei dem Verfahren der hyperspektralen Bildgebung wird eine orts- und wellenlängenaufgelöste Detektion je nach der Fragestellung in den Spektralbereichen des UV (200-400nm), VIS (400-750nm), NIR (750-2500nm) und MIR (2500-10000nm) genutzt, wobei in der Regel mindestens 100 Punkte im Spektrum aufgenommen werden.

Aus der US 2013 004 421 A1 ist eine hyperspektrale Methode der Reflexionsspektroskopie bekannt, mit der die Befruchtungsrate und das Geschlecht eines geschlossenen Vogeleies bestimmt werden können. Zur Auswertung der erhaltenen Spektren wird ein neuronales Netzwerk trainiert und das erhaltene Testspektrum mit einer Spektrenbibliothek verglichen.

Aus der CN 104 316 473 A ist eine Methode des hyperspektralen Transmissionsimaging im Spektralbereich von 400 bis 1000 nm am geschlossenen Hühnerei bekannt. Bei der Auswertung der registrierten Transmissionsspektren werden mathematische Modelle verwendet, die auf der Diskriminanzanalyse mit kleinsten partiellen Quadraten, PLSDA (partial least squares discriminant analysis) oder auf nichtlinearen Algorithmen wie künstlichen neuronalen Netzen, ANN (artificial neural network) oder der Support Vector Machine, SVM (support vector machine) basieren. Nachteilig bei diesem Verfahren ist, das die höchste Prognosegenauigkeit erst am Inkubationstag 10 erreicht wird, wenn der Spektralbereich auf 600 bis 900nm reduziert und eine Region in der Mitte des Eies ausgewählt wurde sowie die Klassifizierung der Spektren basierend auf einem neuronalen Netzwerk erfolgte.

Durch Göhler, D. et.al. ist aus der Poultry Science 96:1-4 (2017) "In-ovo sexing of 14-day-old chicken embryos by pattern analysis in hyperspectral images" ein optisches Verfahren zur Geschlechtsanalyse von Hühnerembryonen mit geschlechtsspezifischer Federfärbung im geschlossenen Hühnerei innerhalb der Inkubationstage 11 bis 14 und somit zu einem erst sehr späten Zeitpunkt nach der Befruchtung des Hühnereies bekannt.

Aus der WO 2016 131 124 A1 ist ein Verfahren und eine Vorrichtung zur Charakterisierung von ungeschlüpften Vögeln bekannt, wobei die erhaltenen Charakteristika auch die Befruchtungsrate und das Geschlecht beinhalten. Die Datenaufnahme erfolgt mit einem Imaging System, die Auswertung der Daten erfolgt mit einem Merkmalsextraktor und einer nachgeschalteten Klassifikation.

Allerdings ist bekannt, dass am Tag 0 nach der Fertilisation der Unterschied zwischen männlichen und weiblichen Eiern allein auf der unterschiedlichen Größe der Geschlechtschromosomen W und Z beruht. So zeigt die DNA im Spektralbereich von 900 bis 1700nm keine Absorptionen, sodass die in diesem Verfahren offenbarte Klassifizierung auf nicht beschriebene Unterschiede in den Eikomponenten Eigelb, Eiweiß und Eischale basiert. Aus diesem Grund wird die Robustheit solch einer Klassifizierung am Tag 0 aufgrund der hohen natürlichen Variabilität der Eier, z.B. durch jahreszeitliche Änderungen oder Änderungen im Futter, dem Alter der Tiere oder einer variierenden Schalendicke, als sehr anfällig eingeschätzt.

Auch bekannt aus der DE 10 2012 016 911 B3 Verfahren und eine Anordnung zur Detektion von Keimscheiben in befruchteten unbebrüteten Geflügeleiern. Das Verfahren wird mit einer Anordnung durchgeführt, die zumindest eine Lichtquelle aufweist, deren Spektrum im Wellenlängen-Bereich zwischen 350 nm und 520 nm liegt. Zudem ist mindestens eine Einrichtung zur Aufnahme eines Eis in eine pollängsachsige Ausgangslage vorhanden, bei der das Eidotter des Eies zumindest über einen der Pole von der Lichtquelle aus bestrahlt wird, und eine Steuereinrichtung, die mit der Einrichtung zur Aufnahme eines Eies und einer Detektionseinrichtung in Verbindung steht. Eien Detektionseinrichtung ist zur bildartigen Aufnahme des bestrahlten Eidotters und der befruchteten Keimscheibe ausgebildet, wobei das Bild der Keimscheibe im Vergleich zum Bild des Eidotters (9) auf der Detektionseinrichtung als Fleck vorhanden ist. Eine Auswerteeinrichtung wertet die mittels eines Bildverarbeitungssystems aus dem detektierten Bild der Keimscheibe die Lage der Keimscheibe innerhalb des Eies aus und gibt über eine Ausgabeeinheit mit Display zur Orts-Erkennung der Keimscheibe als kontrastierender Fleck gegenüber dem Bild des Eidotters auf einem Ausgabebild (23) aus.

Und auch bekannt aus der DE 10 2010 006 161 B3 ist ein Verfahren zur Bestimmung des Geschlechts von befruchteten und nicht bebrüteten Vogeleiern, wobei eine Sonde zur Messung eines Spektrums durch ein Loch in der Eischale hindurch in Richtung zur Keimscheibe mit Keimscheibenzellen geführt wird. Die Verfahrensschritte sind: Positionierung der Sonde im Bereich der Keimscheibe, spektroskopische in-ovo Charakterisierung der Keimscheibenzellen, Erkennung des Geschlechts durch eine automatische Klassifizierung von Spektren, wobei als Sonde ein optischer Kristall eingesetzt wird, mit dem eine schnelle und rückwirkungsfreie Aufnahme eines Infrarot- und/oder Nahinfrarotspektrums bei Nutzung einer abgeschwächten Totalreflexion innerhalb des optischen Kristalls durch ein evaneszentes Feld im Bereich der Keimscheibe durchgeführt wird.

Nachteilig bei den beschriebenen Methoden, die auf der hyperspektralen Bildgebung basieren ist, dass alle natürlich variierenden Ei-Bestandteile im jeweiligen Inkubationsstadium, und damit auch die nicht geschlechtsspezifischen Komponenten, zur Geschlechtsbestimmung in die Auswertung eingehen. Ein weiterer Nachteil der genannten Verfahren besteht darin, dass eine Geschlechtsbestimmung mit einer hohen und stabilen Prognosegenauigkeit nur bei Braunleger-Hühnern möglich ist (in-ovo Farbsexen) und/oder erst am Bruttag 10 bis 14, also in einem späten Fetalstadium, mit sicher vorliegenden Schmerzempfinden des Embryos, durchgeführt werden kann.

Die Aufgabe der Erfindung besteht in der Angabe eines Verfahrens zur in-ovo Fertilisations- und Geschlechtsbestimmung am geschlossenen Ei, mit dem zugleich eine in-ovo Fertilisations- und Geschlechtsbestimmung am geschlossenen Ei realisiert werden kann, die Fertilisations- und Geschlechtsbestimmung während des Bebrütungszeitraumes ermöglicht werden kann und eine hohe Genauigkeit der Befruchtungsraten und Geschlechtsbestimmung ermöglicht wird.

Die Aufgabe wird durch die in den Ansprüchen angegebene Erfindung gelöst. Vorteilhafte Ausgestaltungen der Erfindung sind Gegenstand der Unteransprüche.

Erfindungsgemäß bezieht sich das erfindungsgemäße Verfahren auf die in-ovo Fertilisations- und Geschlechtsbestimmung am geschlossenen Ei, umfassend die Verfahrensschritte
- Positionierung eines geschlossenen Eies,
- Durchleuchtung und/oder Beleuchten des Eies mittels mindestens einer Lichtquelle mit Licht im Spektrum des sichtbaren und/oder nahinfraroten Lichtes,
- Aufnahme eines Bildes vom durchleuchteten und/oder beleuchteten geschlossenen Ei mittels eines optischen Kamerasystems und Auswertung der erfassten Daten des erzeugten Bildes mittels einer Auswerteinheit,
- Berechnung der Position des im Ei befindlichen kardiovaskulären Systems aus den ausgewerteten Daten des Kamerabildes mittels der Auswerteeinheit,
- Einstellung einer Detektionseinheit über der berechneten Position des kardiovaskulären Systems mittels einer Positioniereinheit,
- Anregung des im kardiovaskulären System befindlichen Blutes mittels einer Anregungseinheit,
- Detektion und Selektion der blutspezifischen Absorptionsspektren und der blutfremden Absorptionsspektren mittels einer Erfassungseinheit,
- Bestimmung der Fertilisation, wobei nach der Feststellung der Fertilisation die nachfolgenden Verfahrensschritte durchgeführt werden,
- Kompensation der Spektren mit blutfremden Informationen durch ein Kompensationsverfahren mittels der Auswerteeinheit,
- Klassifizierung der blutspezifischen Absorptionsspektren mittels eines multiplen Klassifizierungsverfahrens zur Bestimmung des Geschlechtes.

Vorteilhafterweise wird, ausgehend von der Lage des spitzen und stumpfen Pols zueinander, das geschlossene Ei im Wesentlichen horizontal oder im Wesentlichen vertikal mit dem spitzen Pol nach oben positioniert.

Ebenfalls vorteilhafterweise wird die Durchleuchtung durch die Lichtquelle unterhalb, oberhalb und/oder seitlich des positionierten Eies realisiert.

Außerdem vorteilhaft ist, wenn als Lichtquelle mindestens eine LED, eine Fluoreszenzlampe, ein breitbandiger Superkontinuum-Weißlicht Laser, ein durchstimmbarer Laser und/oder eine Halogenlampe eingesetzt wird.

Vorteilhafterweise wird eine Lichtquelle mit einem Spektralbereich von UV-Licht bis NIR-Licht eingesetzt, wobei besonders vorteilhaft eine Lichtquelle mit einem Spektralbereich von 350 bis 900 nm eingesetzt wird.

Ebenfalls vorteilhaft ist, wenn zwischen der Lichtquelle und dem Ei ein Filter eingesetzt wird.

Vorteilhaft ist es auch, wenn die Lichtquelle gleichzeitig als Anregungseinheit eingesetzt wird.

Des Weiteren ist vorteilhaft, wenn die Berechnung der Positionsdaten durch Segmentierung des erzeugten Kamerabildes durchgeführt wird.

Ebenfalls vorteilhaft ist, wenn die Erfassung der blutspezifischen und/oder blutfremden Absorptionsspektren mittels eines Spektrometers, Dioden und/oder einer HSI-Kamera und/oder eines FPA-Detektors zusammen mit mindestens einem Objektiv durchgeführt wird.

Weiterhin vorteilhaft ist, wenn zur Kompensation der blutfremden Absorptionsspektren mathematische, physikalische, oder chemische Verfahren eingesetzt werden.

Ebenfalls vorteilhafterweise wird die Kompensation der blutfremden Signale in den Spektren durch Bildung von Differenzen realisiert, wobei die Differenz aus den Spektren mit blutspezifischen und blutfremden Absorptionsspektren und aus Spektren mit blutfremden Absorptionsspektren gebildet wird.

Vorteilhafterweise wird das Verfahren zwischen dem 3. und 4. Bruttag realisiert.

Und auch vorteilhafterweise werden für die Klassifizierung komplementäre geschlechtsspezifische Signale verwendet.

Erfindungsgemäß wird ein Verfahren bereitgestellt, mit dem zugleich eine in-ovo Fertilisations- und Geschlechtsbestimmung am geschlossenen Ei realisiert wird, die Fertilisations- und Geschlechtsbestimmung während des Bebrütungszeitraumes ermöglicht wird und eine hohe Genauigkeit der Befruchtungsraten und Geschlechtsbestimmung ermöglicht wird.

Im Rahmen der vorliegenden Erfindung soll das Verfahren zur in-ovo Fertilisations- und Geschlechtsbestimmung an geschlossenen Eiern durchgeführt werden, wobei unter Ei erfindungsgemäß alle Eier mit einer Schutzhülle zu verstehen sind, die insbesondere eine Kalkschale ist. Insbesondere sind Eier, bei denen das erfindungsgemäße Verfahren zur Anwendung kommt, Vogeleier oder Reptilieneier, wobei besonders Geflügeleier untersucht werden.

Als quasi geschlossenes Ei sollen weiterhin Eier mit einer Pore ≤ 1mm in der Kalkschale und unversehrter äußerer und innerer Schalenmembran verstanden werden, da solche Poren ohne einen zusätzlichen Verschluss die weitere Embryonalentwicklung nicht beeinträchtigen.

Mit dem erfindungsgemäßen Verfahren erfolgt die spektroskopische Geschlechtsbestimmung zu einem sehr frühen Entwicklungsstadium des Eies durch die Analyse von Spektren des embryonalen Blutes, und anschließender Auswertung der aus der Analyse erhaltenen optischen Signale mittels multipler Klassifikationsalgorithmen.

Unter Spektren, die mittels absorptionsspektroskopischer Analysen gewonnen worden sind, den sogenannten Absorptionsspektren, werden alle vom Ei reflektierten, nichtelastisch gestreuten oder transmittierten registrierten Signale verstanden. Die miteinander verbundenen Prozesse Transmission, Absorption und Extinktion, denen jeweils auch eine Bezeichnung für ein Spektrum zugeordnet werden kann, werden hier zusammengefasst als Absorptionsspektrum geführt.

In einem ersten Verfahrensschritt erfolgt die Positionierung der Eier. Dabei wird die Positionierung des kardiovaskulären Systems des Embryos unterhalb der Eischale durch Wendung des Eies aus der Brutschrankposition heraus festgelegt.

Prinzipiell kann die spektroskopische Fertilisations- und Geschlechtsbestimmung in jeder Eiposition durchgeführt werden.

Bevorzugt sind jedoch die horizontale Positionierung des Eies, bei der sich spitzer und stumpfer Pol des Eies horizontal gegenüberliegen, oder die Positionierung des Eies mit dem spitzen Pol nach oben. Bei diesen Positionen des Eies befindet sich das kardiovaskuläre System direkt unter der Eischale, da sich während der ersten Inkubationstage im Ei zwischen den einzelnen Ei-Komponenten durch Stoffwechselprozesse Dichteunterschiede bilden. Diese führen dazu, dass sich der auf dem Dotter anhaftende Embryo einschließlich der extraembryonalen Gefäße bei einer Ei-Drehung immer am höchsten Punkt innerhalb des Eies befindet. Wird das Ei in der Brutposition belassen, wie sie bei regulärem Brutmanagement in einer kommerziellen Brüterei angewendet wird (stumpfer Eipol aufwärts, spitzer Eipol abwärtsgerichtet), dann befindet sich zwischen Embryo einschließlich des embryonalen kardiovaskulären Systems und der Eischale eine Luftkammer.

Eine besonders vorteilhafte Ausführung des erfindungsgemäßen Verfahrens ist die Analyse am geschlossenen spitzen Eipol, da die Wendung der Eier in großer Anzahl in kommerziell genutzten Ei-Horden technisch sehr einfach zu realisieren ist. Ein weiterer Vorteil dieser Positionierung besteht darin, dass die Eischalenfläche, unter der sich der Embryo mit dem Blutgefäßnetz befinden kann, am spitzen Pol durch die stärkere Krümmung der Schale kleiner gegenüber der Eischalenfläche bei horizontaler Eiposition ist. Das führt zu einer sicheren und schnelleren Detektion des kardiovaskulären Systems und kürzeren Motor-Fahrwegen der detektierenden Optik.

Nach der Positionierung des mindestens einen Eies erfolgt die Be- oder Durchleuchtung des Eies mittels einer Lichtquelle mit Licht im Spektrum des sichtbaren und/oder Nahinfraroten Lichtes.

Bei der Beleuchtung des Eies soll im Rahmen der Erfindung die Registrierung der Absorptionspektren vorzugsweise im Transflexionsmodus verstanden werden.

Bei der Durchleuchtung des Eies soll im Rahmen der Erfindung die Registrierung der Absorptionsspektren vorzugsweise im Transmissionsmodus verstanden werden.

Dieser Verfahrensschritt der Be- oder Durchleuchtung des Eies dient zum Auffinden des kardiovaskulären Systems mittels eines optischen Kamerasystems, insbesondere mittels einer Kamera.

Innerhalb des elektromagnetischen Spektrums umfasst das Lichtspektrum alle vom Menschen sichtbaren Anteile des Spektrums, mit Wellenlängen zwischen ca. 400 und ca. 700 nm. Die erfindungsgemäß eingesetzte Lichtquelle kann Licht im Lichtspektrum aussenden. Sofern die optischen Aufnahmesysteme dazu ausgestattet sind, kann die Lichtquelle auch Licht im Bereich der Ultravioletten und/oder Infraroten Spektren aussenden. Entscheidend ist, dass mit dem optischen Kamerasystem ein Auffinden des kardiovaskulären Systems im Ei realisiert wird.

Die Position und Anordnung der Lichtquelle kann prinzipiell von allen Seiten des positionierten Eies erfolgen, beispielsweise unterhalb des Eies, seitlich des Eies oder oberhalb des Eies. Als Lichtquelle wird vorteilhafterweise eine breitbandige Lichtquelle eingesetzt, beispielsweise LED, Fluoreszenzlampen, Gasentladungslampen, breitbandiges Superkontinuum-Weißlichtlaser oder Halogenlampen mit Filtern, wobei die Filter zur Vermeidung starker Wärmestrahlung auf das positionierte Ei dienen.

Während der Be- oder Durchleuchtung des positionierten Eies mittels Lichtquelle erfolgt die Aufnahme eines Bildes mittels eines über dem Ei angeordneten optischen Kamerasystems. Das Kamerasystem registriert ein Bild des Embryos und des kardiovaskulären Systems. Nach der Bildaufnahme erfolgt die Auswertung der Bilddaten, wobei die Positionsdaten des kardiovaskulären Systems ermittelt werden, beispielsweise durch Segmentierung. Diese Positionsdaten werden an eine Zentriereinheit übergeben und dazu verwendet, die Detektionseinheit und im Fall der Transflexionsmethode auch die Anregungseinheit über dem kardiovaskulären System zu positionieren. Kann kein kardiovaskuläres System im Ei detektiert werden, kann das Ei als unbefruchtet aussortiert werden. Damit ist bereits an diesem Verfahrensschritt die Fertilisationsbestimmung erfolgt.

Die Lichtquelle für die Be- oder Durchleuchtung des Eies kann gleichzeitig die Anregungseinheit darstellen. Dies ist dann der Fall, wenn die Methode der Transmission angewendet wird, bei der das Ei von unten und/oder seitlich zur Erfassung der Positionsdaten des kardiovaskulären Systems durch die Lichtquelle durchleuchtet und damit gegenüberliegend oder in einem bestimmten Winkel zum optischen Kamerasystem und der Detektionseinheit angeordnet ist. Bei dieser Anordnung stellt die unter dem Ei und/oder seitlich vom Ei angeordnete Lichtquelle gleichzeitig die Anregungseinheit dar.

Im Gegensatz dazu kann das Ei zur Erfassung der Positionsdaten des einerseits von unten durchleuchtet werden und zudem eine Anregungseinheit oberhalb des Eies angeordnet sein, die ebenfalls eine Lichtquelle darstellt und bei der die Strahlung der Anregungseinheit vom zur Detektionseinheit reflektiert und detektiert wird (Transflexion).

Prinzipiell kann das Auffinden des kardiovaskulären Systems im Ei direkt mit einer HSI-Kamera oder einem Focal-Plane Array (FPA) Detektor erfolgen. Hierzu ist es vorteilhaft, wenn eine HSI-Kamera mit einem Weitwinkel-Objektiv und/oder einen großen Arraydetekor eingesetzt wird, um die gesamte Eioberfläche, an der sich der Embryo und das kardiovaskuläre System befinden, registrieren zu können. Die Verarbeitung des dreidimensionalen Datensatzes der HSI-Kamera oder des FPA-Detektors in der Auswerteeinheit ist jedoch zeitlich aufwendiger als die Aufnahme eines RGB- oder Monochrom-Images, weshalb die Kopplung eines solchen Visionssystems mit einer HSI-Kamera oder eines FPA-Detektors eine technisch vorteilhafte Lösung darstellt.

Nach der Positionierung der Detektionseinheit und der Anregung des im kardiovaskulären System befindlichen Blutes mittels einer Anregungseinheit erfolgt die Detektion und Selektion der blutspezifischen und blutfremden Absorptionsspektren, wobei die Anregung des Blutes mittels des durch das kardiovaskuläre System transmittiertem Anregungslicht oder durch die Blutgefäße transreflektiertem Anregungslicht erfolgt.

Die detektierten Absorptionsspektren lassen sich in zwei Gruppen a) und b) unterschieden:
(a) Spektren, die sich aus blutspezifischen Informationen und blutfremden Informationen zusammensetzen, und
(b) Spektren, die ausschließlich blutfremde Informationen aufweisen.

Möglich ist aber auch, dass Spektren nur aus blutspezifischen Informationen bestehen können.

Die Absorptionsspektren des Blutes werden in den Spektralbereichen von UV- bis NIR-Licht, bevorzugt jedoch im Spektralbereich von 350 bis 900 nm, am geschlossenen Ei registriert und bei der sich anschließenden Auswertung der Absorptionsspektren die optischen Signale aller das kardiovaskuläre System umgebenden und damit blutfremden Eibestandteile, einschließlich der Eischale, durch optische, biochemische und/oder mathematische Kompensation entfernt.

Zum Erreichen einer verbesserten Störsicherheit und einer höheren Genauigkeit bei der Geschlechtsidentifizierung kann in einer besonders vorteilhaften Ausgestaltung des Verfahrens während oder nach der Erfassung des kardiovaskulären Systems eine Messeinheit zur Bestimmung des Durchmessers des erfassten kardiovaskulären Systems durch eine Pore, die ausschließlich in die Kalkschale eingebracht wird und einen Durchmesser ≤ 1mm aufweist, eingebracht werden.

Der äußerst geringe Durchmesser der Pore von ≤ 1 mm führt dazu, dass nach Entfernen der Messeinheit die Pore von selbst wieder ausheilt und sich verschließt. Das Ei selbst wird durch das Einbringen der Pore ausschließlich in die Kalkschale daher nicht als "geöffnetes Ei" angesehen, sodass auch der embryonale Entwicklungsverlauf nicht beeinflusst wird, wie dies bei der Öffnung des Eies aus dem Stand der Technik bekannt ist.

Der Vorteil des besonders kleinen Loches von ≤ 1mm liegt darin, dass die Messeinheit, beispielsweise in Form einer optischen Faser mit einem Durchmesser kleiner 1 mm durch die Pore bis auf die unversehrt gebliebene Schalenmembran geführt werden kann, so dass optische Effekte der Kalkschale (z.B. Fluoreszenz, Rayleigh-Streuung) nicht miterfasst werden. Ein weiterer Vorteil liegt zudem in der damit möglichen Registrierung von Absorptionsspektren und/ oder Fluoreszenzlicht des Blutes zur Geschlechtsbestimmung. Bei der vollständig geschlossenen Kalkschale würde das Fluoreszenzlicht des Blutes von der Fluoreszenz und Absorption der Kalkschale überlagert.

In einer vorteilhaften Ausführung des Verfahrens erfolgt die Detektion des transmittierten Anregungslichtes mittels einer HSI-Kamera oder FPA-Detektors, mit welcher die Messung eines spektralen, dreidimensionalen Bildstapels möglich ist. Dabei wird die HSI-Kamera oder der FPA-Detektor entsprechend den Positionsdaten des Kamerasystems so positioniert, dass die x*y Imaging-Fläche sowohl Positionen innerhalb (a) und außerhalb (b) der Blutgefäße einschließt, wobei jedem Bildpunkt der x*y Imaging-Fläche ein Absorptionsspektrum an dieser Bildposition zugeordnet werden kann.

Eine weiter bevorzugte Ausführung ist die Detektion des Anregungslichtes mittels einer Detektorzeile, einer HSI-Kamera oder eines FPA-Detektors, bei der nur eine Zeile x oder Spalte y registriert wird, wobei diese Zeile/Spalte ebenso Positionen innerhalb (a) und außerhalb (b) der Blutgefäße einschließen muss. Diese Ausführung hat den technischen Vorteil, dass ein schnellerer Durchsatz bei der Geschlechtsbestimmung bei technisch einfacherer Konstruktion durch Motoren-Fahrwege ausschließlich in eine Richtung realisiert wird.

Aus dem Bild, der Zeile/Spalte oder einzelnen Messpunkten werden nach der Aufnahme die blutspezifischen Absorptionsspektren, welche geschlechtsspezifische Informationen des embryonalen Blutes enthalten (a) von denen der blutfremden Bestandteile (Eiweiß, Eigelb, innere und äußere Schalenmembran, Kalkschale) außerhalb der Blutgefäße (b) selektiert.

Die Kompensation blutfremder Absorptionsspektren erfolgt durch Selektion von spektralen Signalen des Blutes aus allen detektierten Absorptionsspektren des Eies.

Die Detektion der Absorptionsspektren erfolgt innerhalb des erfindungsgemäßen Verfahrens derart, dass mindestens ein, bevorzugt jedoch mehrere Absorptionsspektren der Gruppe (a) und mindestens ein, bevorzugt jedoch mehrere Absorptionsspektren der Gruppe (b) detektiert werden.

Einzelpunktmessungen innerhalb der Position der Blutgefäße (Gruppe (a)) und außerhalb der Position der Blutgefäße (Gruppe (b)) werden durchgeführt, indem eine Mikroskopoptik in ein Blutgefäß und außerhalb eines Blutgefäßes fokussiert wird und die Spektren mit einem Spektrometer detektiert werden.

Aus der Gesamtzahl der detektierten Absorptionsspektren werden die Spektren selektiert, welche neben den blutfremden Absorptionen der Bestandteile des Eies (Eigelb, Eiweiß, Eimembranen und Kalkschale) die Absorptionsspektren des embryonalen Blutes aufweisen.

Die Gruppe der nichtselektierten Absorptionsspektren enthalten ausschließlich spektrale Signale aller weiteren Bestandteile des Eies, jedoch keine Informationen über das embryonale Blut. Durch beispielsweise mathematische Operationen zwischen beiden Gruppen, beispielsweise mittels Differenzbildungen, wird die Kompensierung der Absorptionssignale der die Blutgefäße umgebenden weiteren Bestandteile des Eies ermöglicht.

Die Klassifizierung der blutspezifischen und damit auch geschlechtsspezifischen Absorptionsspektren erfolgt mittels einem multiplen Klassifizierungsverfahren, wobei vorteilhafterweise nach der Erfassung der geschlechtsspezifischen Absorptionsspektren und der Datenvorbehandlung der blutspezifischen Absorptionsspektren ein multiples Klassifizierungsverfahren mit mindestens zwei unterschiedlichen Verfahren der Datenvorbehandlung der Spektren und des der jeweiligen Datenvorbehandlung zugeordneten Verfahrens zur Klassifizierung durchgeführt wird.

Mit dem erfindungsgemäßen Verfahren werden folgende technische Wirkungen und Vorteile gegenüber dem Stand der Technik erreicht.

Überraschenderweise wurde festgestellt, dass die Absorptionsspektren des Blutes geschlechtsspezifisch sind und während der Entwicklung des Embryos auch bleiben. Damit wird es erstmalig möglich, unter Berücksichtigung der Kompensation der weiteren Bestandteile des Eies die Fertilisationsbestimmung und Geschlechtsbestimmung am geschlossenen Ei mittels Absorption der blutspezifischen Spektren durchzuführen. Das erfindungsgemäße Verfahren nutzt zur Geschlechtsbestimmung kleine, aber geschlechtsspezifische Unterschiede in der Molekülstruktur des Hämoglobins. Es beruht dabei nicht nur darauf Signale, die vom zentralen Strukturelement, dem Porphyrin-Ring, herrühren, zu erfassen, sondern vielmehr auch auf Informationen über innermolekulare Verschiebungen der Elektronendichte und Energiezustände durch die Substituenten bzw. Seitengruppen.

Ein wesentlicher Vorteil des erfindungsgemäßen Verfahrens ist es, dass die Fertilisations- und Geschlechtsbestimmung am geschlossenen Ei erfolgt. Dadurch kann das aus dem Stand der Technik bekannte aufwendige Öffnung und Schließen des Eies entfallen, was zu wesentlich geringeren Verfahrenskosten führt. Eine Vorbehandlung der Eierschale, um diese für eine nachfolgende Untersuchung transparenter zu machen, ist ebenfalls nicht notwendig. Insbesondere bleibt durch die am geschlossenen Ei durchgeführte Fertilisations- und Geschlechtsbestimmung die Embryonalentwicklung unbeeinflusst, was zudem zu verbesserten Schlupfraten führt.

Durch die erstmalige Nutzung von Absorptionsspektren allein aus Blutgefäßen des geschlossenen Eies werden intensive, geschlechtsspezifische optische Signale aus dem embryonalen Blut erhalten. Gegenüber bisher bekannten Ramanspektroskopischen Verfahren und Untersuchungen wird dadurch eine wesentlich verbessere Signalqualität mit geringer Fehlerquote bei der Geschlechtsbestimmung erreicht.

Zudem wird erfindungsgemäß durch die Kompensation von starken Absorptionen der die Blutgefäße umgebenden Eibestandteile des Eies, welche keinen Beitrag zur Fertilisations- und Geschlechtsanalyse liefern, eine hohe Klassifizierungsgenauigkeit erreicht. Die Klassifikation ist dadurch stabil und unabhängig von der natürlichen Variabilität der Eier und der Variabilität im Inkubationsprozess.

Gegenüber anderen aus dem Stand der Technik bekannten Verfahren besteht ein weiterer wesentlicher Vorteil der Erfindung darin, dass insbesondere die Geschlechtsbestimmung zu einem sehr frühen Stadium und über einen langen Zeitraum nach der Inkubation zuverlässig und mit sehr hoher Genauigkeit erfolgt, da die geschlechtsspezifischen Absorptionsspektren des embryonalen Blutes im Gegensatz zu den Informationen der weiteren Bestandteile des Eies während der Bebrütung konstant bleiben. Dies ist bei den anderen Bestandteilen des Eies, wie beispielsweise dem Eigelb oder dem Eiweiß, eben gerade nicht der Fall.

Zudem ist es erfindungsgemäß möglich, die Messung der geschlechtsspezifischen Absorptionssignale am geschlossenen Ei in Kombination mit geschlechtsspezifischen Fluoreszenzsignalen durchzuführen. Hierbei ist zu berücksichtigen, dass die Fluoreszenz der Eischale entweder mathematisch durch registrierte Referenzspektren der Eischale oder auch durch optische oder chemische Fluoreszenzlöschung vorteilhafterweise kompensiert werden muss.

Aus den genannten verschiedenen Positionierungen, Lichtquellen für die Kamera, Anregungsquellen für die Absorption und möglicherweise für die Fluoreszenz und Detektoren ergeben sich zudem eine Vielzahl an optischen Kombinationsmöglichkeiten für dieses Verfahren, sodass trotz der Variabilität der Anordnung der einzusetzenden Komponenten gleichbleibend und zuverlässige Analyseergebnisse erzielt werden.

Ein weiterer Vorteil des erfindungsgemäßen Verfahrens ist es, dass für die Klassifizierung der blutspezifischen Absorptionsspektren komplementäre geschlechtsspezifische Signale verwendet werden können. Dies hat den Vorteil, dass mittels interner Referenzierung die geschlechtsspezifischen Unterschiede herausgehoben werden, wobei die interne Referenzierung zum Beispiel durch Verhältnisbildung der Intensität (Integral) im kürzerwelligen Spektralbereich zur Intensität im längerwelligen Spektralbereich eine interne Referenzierung erfolgt.

Nachfolgend wird die Erfindung anhand von zwei Ausführungsbeispielen näher erläutert. Dabei zeigen die
- Abbildung 1: In-ovo Mittelwert-Absorptionsspektren von embryonalem Blut, gemessen in geschlossenen Hühnereiern nach 3,5 Tagen Bebrütung, nach Differenzbildung mit den weiteren Bestandteilen des geschlossenen Eies, die
- Abbildung 2: den optischen Aufbau im Transmissionsmodus zur Messung der geschlechtsspezifischen Absorption des embryonalen Blutes mittels einer Spektrometer-gekoppelten Faseroptik bei horizontaler Lagerung des Eies, die

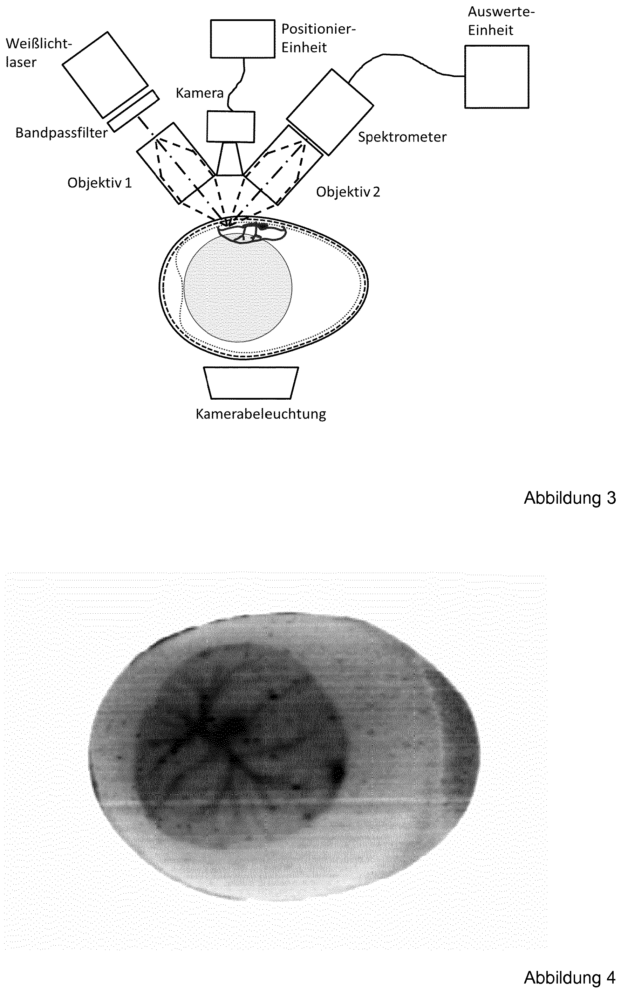

- Abbildung 3: den optischen Aufbau im Transflexionsmodus zur Messung der geschlechtsspezifischen Absorption des embryonalen Blutes, und die
- Abbildung 4: eine HSI-Bildaufnahme eines 3,5 Tage angebrüteten Hühnereies mit embyonalen kardiovaskulären Strukturen

### Ausführungsbeispiel 1

Ein Hühnerei wird nach 3,5 Inkubationstagen dem Brutschrank entnommen, aus der Brutposition um 90° gewendet und über einer Halogenlampe, welche eine regelbare Leistung von 0 - 50W und ein Emissionsspektrum im Spektralbereich zwischen 400 und 900 nm aufweist, in einem Abstand von ca. 4 cm positioniert. In der oberen Hälfte der Eikrümmung des zu messenden Eies befindet sich eine Blende mit einer elliptischen Öffnung, welche ein direktes Auftreffen von Anregungslicht auf den Detektor und die damit verbundene Sättigung der Detektoreinheit verhindert. Nach Einschalten der Halogenlampe wird das Ei durchleuchtet und der Embryo mit dem Blutgefäßnetz sichtbar. Die Positionierung des Eies erfolgte nun derart, dass sich innerhalb der Blendenöffnung mindestens teilweise der Embryo und die extraembryonalen Blutgefäße befinden. Für die Erfassung der Positionen der Herzstruktur innerhalb des Embryos und des extraembryonalen Blutgefäßnetzes wird der grüne Kanal einer RGB-Kamera genutzt. Das erhaltene Kamerabild wird segmentiert und die Positionsdaten an ein kundenspezifisches Mikrorobotersystem übergeben. Dieses System transportiert nun ein faseroptisches System der Fa. Grintech, bestehend aus einer multimodalen Faser und einer Mikro-Grinlinse (Durchmesser d=0,5mm) entsprechend den Positionsdaten zum embryonalen Herzen, wobei die Positionierung in einem Arbeitsabstand von 0,5mm zur Eierschale und in einem 90° Winkel zur Eischalentangente erfolgte. Die Faser des faseroptischen Systems wird mit einem UV/VIS-Spektrometer der Fa. Ocean Optics gekoppelt und ein Absorptionsspektrum im Spektralbereich zwischen 480 und 900 nm registriert. Eine zweite Positionierung der Faseroptik mit anschließender Registrierung eines Spektrums erfolgt innerhalb der Blendenöffnung, jedoch außerhalb aller segmentierten Blutgefäßpositionen und der Herzposition. Nach Abschluss beider Messungen werden in der Auswerteeinheit die Differenzspektren gebildet und einer multiplen Datenvorbehandlung unterzogen. Es werden somit Mittelwertdifferenzspektren für männliches und weibliches embryonales Blut erhalten.

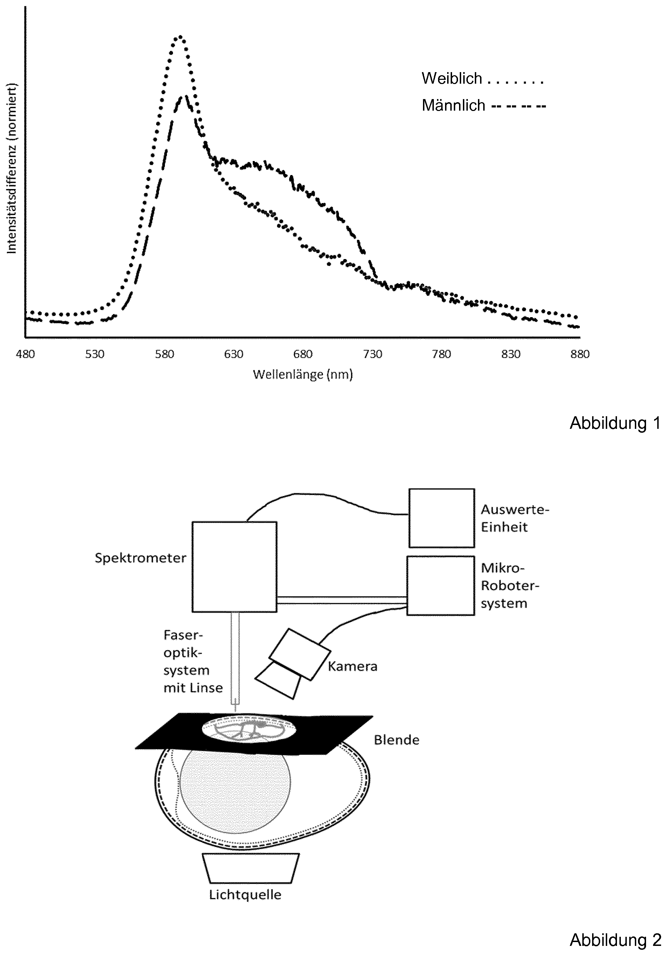

In Abbildung 1 ist deutlich zu erkennen, dass es bei ca. 610 nm einen Schnittpunkt zwischen dem männlichen und weiblichen Mittelwertspektrum gibt und damit wellenlängenabhängige geschlechtsspezifische Intensitätsumkehr vorliegt.

Nach interner Referenzierung der wellenlängenabhängigen, geschlechtsspezifischen Intensitäten konnte die abschließende Geschlechtsbestimmung anhand der geschlechtsspezifischen Signale im Wellenlängenbereich 500-900nm mittels multipler Klassifikationsalgorithmen erfolgen.

### Ausführungsbeispiel 2

Ein Hühnerei wird nach 3,5 Inkubationstagen dem Brutschrank entnommen, aus der Brutposition um 90° gewendet und über einer LED-Lampe, welche Licht im Spektralbereich zwischen 400 und 730 nm aufweist, in einem Abstand von ca. 1 cm positioniert. Nach Einschalten der LED-Lampe wird das Ei durchleuchtet und der Embryo mit dem Blutgefäßnetz sichtbar. Für die Erfassung der Positionen der Herzstruktur innerhalb des Embryos und des extraembryonalen Blutgefäßnetzes wird der grüne Kanal einer RGB-Kamera genutzt. Das erhaltene Kamerabild wird segmentiert und die Positionsdaten werden an ein kundenspezifisches Mikrorobotersystem übergeben. Mit diesen Positionsdaten wird ein Objektiv 1 (NA=0.4, WD=20mm, Vergrößerung 20-fach, Fa. Mitutoyo) so positioniert, dass das Anregungslicht eines Superkontinuum-Weißlichtlasers (Fa. NKT Photonics), gefiltert mit einem Bandpassfilter im Spektralbereich 450-600nm, in die embryonale Herzstruktur fokussiert wird. Mit den Positionsdaten des Robotersystems wird ein zweites Objektiv ebenfalls so über der Herzstruktur positioniert, dass das transflektierte, rückgestreute Signal gesammelt und in Freistrahloptik direkt in den Eingangsspalt eines UV/VIS-Spektrometers (FLAME, Fa. Ocean Optics) fokussiert wird, in welchem die Registrierung der Absorptionssignale erfolgt. Eine zweite Positionierung der beiden Mikroskop-Objektive mit anschließender Registrierung eines Spektrums erfolgt außerhalb aller segmentierten Blutgefäßpositionen und der Herzposition. Nach Abschluss beider Messungen werden in der Auswerteeinheit die Differenzspektren gebildet und einer multiplen Datenvorbehandlung unterzogen. Die abschließende Geschlechtsbestimmung erfolgt anhand der geschlechtsspezifischen Signale im Emissions-Spektralbereich mittels multipler Klassifikationsalgorithmen.

## Patentansprüche

1. Verfahren zur in-ovo Fertilisations- und Geschlechtsbestimmung am geschlossenen Ei, umfassend die Verfahrensschritte
a) Positionierung eines geschlossenen Eies,
b) Durchleuchtung und/oder Beleuchten des Eies mittels mindestens einer Lichtquelle mit Licht im Spektrum des sichtbaren und/oder nahinfraroten Lichtes,
c) Aufnahme eines Bildes vom durchleuchteten und/oder beleuchteten geschlossenen Ei mittels eines optischen Kamerasystems und Auswertung der erfassten Daten des erzeugten Bildes mittels einer Auswerteinheit,
d) Berechnung der Position des im Ei befindlichen kardiovaskulären Systems aus den ausgewerteten Daten des Kamerabildes mittels der Auswerteeinheit,
e) Einstellung einer Detektionseinheit mittels Positioniereinheit über der berechneten Position des kardiovaskulären Systems,
f) Anregung des im kardiovaskulären System befindlichen Blutes mittels einer optischen Anregungseinheit mindestens durch die innere und äußere Schalenmembran sowie Kalkschale des Eies,
g) Detektion und Selektion der blutspezifischen Absorptionsspektren und der blutfremden Absorptionsspektren mindestens durch die innere und äußere Schalenmembran sowie Kalkschale des Eies mittels einer Erfassungseinheit,
h) Bestimmung der Fertilisation, wobei nach der Feststellung der Fertilisation die nachfolgenden Verfahrensschritte durchgeführt werden,
i) Kompensation der Spektren mit blutfremden Informationen durch ein Kompensationsverfahren mittels der Auswerteeinheit, wobei die Kompensation der blutfremden Signale in den Spektren durch Bildung von spektroskopischen Differenzen realisiert wird, wobei die Differenz aus den Spektren mit blutspezifischen und blutfremden Absorptionsspektren, einschließlich der Absorptionsspektren der inneren und äußeren Schalenmembran sowie der Kalkschale, und aus Spektren mit blutfremden Absorptionsspektren, einschließlich der Absorptionsspektren der inneren und äußeren Schalenmembran sowie der Kalkschale, gebildet wird,
j) Klassifizierung der blutspezifischen Absorptionsspektren mittels eines multiplen Klassifizierungsverfahrens zur Bestimmung des Geschlechtes.

2. Verfahren nach Anspruch 1, bei dem, ausgehend von der Lage des spitzen und stumpfen Pols zueinander, das geschlossene Ei im Wesentlichen horizontal oder im Wesentlichen vertikal mit dem spitzen Pol nach oben positioniert wird.

3. Verfahren nach Anspruch 1 oder 2, bei dem die Durchleuchtung durch die Lichtquelle unterhalb, oberhalb und/oder seitlich des positionierten Eies realisiert wird.

4. Verfahren nach mindestens einem der vorhergehenden Ansprüche, bei dem als Lichtquelle mindestens eine LED, eine Fluoreszenzlampe, ein breitbandiger Superkontinuum-Weißlicht-Laser, ein durchstimmbarer Laser und/oder eine Halogenlampe eingesetzt werden.

5. Verfahren nach mindestens einem der vorhergehenden Ansprüche, bei dem eine Lichtquelle mit einem Spektralbereich von UV-Licht bis NIR-Licht eingesetzt wird, besonders vorteilhaft mit einem Spektralbereich von 350 bis 900 nm.

6. Verfahren nach mindestens einem der vorhergehenden Ansprüche, bei dem zwischen der Lichtquelle und dem Ei ein Filter eingesetzt wird.

7. Verfahren nach mindestens einem der vorhergehenden Ansprüche, bei dem die Lichtquelle gleichzeitig als Anregungseinheit eingesetzt wird.

8. Verfahren nach mindestens einem der vorhergehenden Ansprüche, bei dem die Berechnung der Positionsdaten des kardiovaskulären Systems durch Segmentierung des erzeugten Kamerabildes durchgeführt wird.

9. Verfahren nach mindestens einem der vorhergehenden Ansprüche, bei dem die Erfassung der blutspezifischen und/oder blutfremden Absorptionsspektren mittels eines Spektrometers, Dioden und/oder einer HSI-Kamera und/oder eines FPA Detektors zusammen mit mindestens einem Objektiv durchgeführt wird.

10. Verfahren nach mindestens einem der vorhergehenden Ansprüche, bei dem während oder nach der Erfassung des kardiovaskulären Systems eine Messeinheit zur Bestimmung des Durchmessers des kardiovaskulären Systems ausschließlich durch eine Pore in der Kalkschale mit einem Durchmesser von ≤ 1mm eingebracht wird.

11. Verfahren nach mindestens einem der vorhergehenden Ansprüche, bei dem die Geschlechtsbestimmung mittels Absorptionssignalen und/oder Fluoreszenzsignalen durch eine Pore ≤ 1mm des Eies realisiert wird.

12. Verfahren nach mindestens einem der vorhergehenden Ansprüche, bei dem für die Klassifizierung komplementäre geschlechtsspezifische Signale verwendet werden.

13. Verfahren nach Anspruch 12, bei dem die Klassifizierung mittels Kombination / logischer Verknüpfung der komplementären geschlechtsspezifischen Signale durch mathematische Verfahren, besonders vorteilhaft durch Quotientenbildung, durchgeführt wird.

14. Verfahren nach mindestens einem der vorhergehenden Ansprüche, wobei das Verfahren zwischen dem 3. und 4. Bruttag realisiert wird.

## Claims

1. Method for the in-ovo determination of fertilization and sex on a closed egg, comprising the method steps of
a) positioning a closed egg,
b) transilluminating and/or illuminating the egg by means of at least one light source using light in the visible and/or near-infrared light spectrum,
c) capturing an image of the transilluminated and/or illuminated closed egg by means of an optical camera system and evaluating the recorded data from the generated image by means of an evaluation unit,
d) calculating the position of the cardiovascular system located in the egg from the evaluated data from the camera image by means of the evaluation unit,
e) adjusting a detection unit above the calculated position of the cardiovascular system by means of a positioning unit,
f) exciting the blood located in the cardiovascular system at least through the inner shell membrane, the outer shell membrane, and the calcareous shell of the egg by means of an optical excitation unit,
g) detecting and selecting the blood-specific absorption spectra and the non-blood-related absorption spectra at least through the inner shell membrane, the outer shell membrane, and the calcareous shell of the egg by means of a recording unit,
h) determining whether the egg has been fertilized, wherein after fertilization has been established, the following method steps are performed,
i) compensating for the spectra with non-blood-related information through a compensation method by means of the evaluation unit, wherein the non-blood-related signals in the spectra are compensated for by forming spectroscopic differentials, wherein the differential is formed from the spectra with blood-specific and non-blood-related absorption spectra, including the absorption spectra of the inner shell membrane, the outer shell membrane, and the calcareous shell, and from spectra with non-blood-related absorption spectra, including the absorption spectra of the inner shell membrane, the outer shell membrane, and the calcareous shell,
j) classifying blood-specific absorption spectra by means of a multiple classification method to determine the sex.

2. Method according to claim 1, wherein starting from the position of the pointed and blunt ends relative to each other, the closed egg is positioned substantially horizontally or substantially vertically with the pointed end pointing upward.

3. Method according to claim 1 or 2, wherein the egg is transilluminated by the light source below, above, and/or to the side of the positioned egg.

4. Method according to at least one of the preceding claims, wherein at least one LED, fluorescent lamp, wideband supercontinuum white light laser, tunable laser, and/or halogen lamp is used as the light source.

5. Method according to at least one of the preceding claims, wherein a light source with a spectral range from UV light to NIR light is used, particularly advantageously with a spectral range of 350 to 900 nm.

6. Method according to at least one of the preceding claims, wherein a filter is inserted between the light source and the egg.

7. Method according to at least one of the preceding claims, wherein the light source is simultaneously used as the excitation unit.

8. Method according to at least one of the preceding claims, wherein the position data of the cardiovascular system are calculated by segmenting the generated camera image.

9. Method according to at least one of the preceding claims, wherein the blood-specific and/or non-blood-related absorption spectra are recorded by means of a spectrometer, diodes, and/or an HSI camera, and/or an FPA detector together with at least one lens.

10. Method according to at least one of the preceding claims, wherein while the cardiovascular system is being recorded or after it has been recorded, a measuring unit for determining the diameter of the cardiovascular system is introduced exclusively through a pore in the calcareous shell with a diameter of ≤ 1 mm.

11. Method according to at least one of the preceding claims, wherein the sex is determined by means of absorption signals and/or fluorescence signals through a pore of ≤ 1 mm of the egg.

12. Method according to at least one of the preceding claims, wherein complementary sex-specific signals are used for classifying the spectra.

13. Method according to claim 12, wherein the spectra are classified by combining/logically linking the complementary sex-specific signals through mathematical methods, particularly advantageously by forming quotients.

14. Method according to at least one of the preceding claims, wherein the method is performed between the 3rd and 4th incubation day.

## Revendications

1. Procédé permettant la fécondation et le sexage *in ovo* d'un œuf fermé, comprenant les étapes de procédé consistant à
a) positionner un œuf fermé,
b) mirer et/ou éclairer l'œuf au moyen d'au moins une source de lumière comportant une lumière dans le spectre de la lumière visible et/ou du proche infrarouge,
c) capturer une image de l'œuf fermé miré et/ou éclairé au moyen d'un système formant caméra optique, et analyser les données de l'image générée détectées au moyen d'une unité d'analyse,
d) calculer la position du système cardiovasculaire présent dans l'œuf à partir des données de l'image de caméra analysées au moyen de l'unité d'analyse,
e) régler une unité de détection, au moyen d'une unité de positionnement, au-dessus de la position calculée du système cardiovasculaire,
f) stimuler le sang présent dans le système cardiovasculaire au moyen d'une unité de stimulation optique, au moins à travers la membrane interne et externe de la coquille ainsi que la coquille calcaire de l'œuf,
g) détecter et sélectionner les spectres d'absorption spécifiques au sang et les spectres d'absorption non liés au sang, au moins à travers la membrane interne et externe de la coquille ainsi que la coquille calcaire de l'œuf, au moyen d'une unité de détection,
h) déterminer la fécondation, dans lequel les étapes de procédé suivantes sont mises en œuvre une fois la fécondation constatée,
i) compenser les spectres comportant des informations non liées au sang par l'intermédiaire d'un procédé de compensation au moyen de l'unité d'analyse, dans lequel la compensation des signaux non liés au sang dans les spectres est réalisée par la formation de différences spectroscopiques, dans lequel la différence est formée à partir des spectres comportant des spectres d'absorption spécifiques au sang et non liés au sang, y compris les spectres d'absorption des membranes interne et externe de la coquille ainsi que de la coquille calcaire, et à partir de spectres comportant des spectres d'absorption non liés au sang, y compris les spectres d'absorption des membranes interne et externe de la coquille ainsi que de la coquille calcaire,
j) classer les spectres d'absorption spécifiques au sang au moyen d'un procédé de classification multiple permettant de déterminer le sexe.

2. Procédé selon la revendication 1, dans lequel, en fonction de la position des bouts pointu et émoussé, l'œuf fermé est positionné sensiblement à l'horizontale ou sensiblement à la verticale avec le bout pointu vers le haut.

3. Procédé selon la revendication 1 ou 2, dans lequel le mirage est réalisé par l'intermédiaire de la source de lumière située en dessous, au-dessus et/ou sur le côté de l'œuf positionné.

4. Procédé selon au moins l'une des revendications précédentes, dans lequel au moins une DEL, une lampe fluorescente, un laser à lumière blanche à supercontinuum à large bande, un laser ajustable et/ou une lampe halogène sont utilisés comme sources de lumière.

5. Procédé selon au moins l'une des revendications précédentes, dans lequel une source de lumière avec un domaine spectral allant de la lumière UV à la lumière proche infrarouge est utilisée, de manière particulièrement avantageuse avec un domaine spectral allant de 350 à 900 nm.

6. Procédé selon au moins l'une des revendications précédentes, dans lequel un filtre est intercalé entre la source de lumière et l'œuf.

7. Procédé selon au moins l'une des revendications précédentes, dans lequel la source de lumière est utilisée simultanément comme unité de stimulation.

8. Procédé selon au moins l'une des revendications précédentes, dans lequel le calcul des données de position du système cardiovasculaire est mis en œuvre par l'intermédiaire d'une segmentation de l'image de caméra générée.

9. Procédé selon au moins l'une des revendications précédentes, dans lequel la détection des spectres d'absorption spécifiques au sang et/ou non liés au sang est mise en œuvre au moyen d'un spectromètre, de diodes et/ou d'une caméra HSI et/ou d'un détecteur FPA, conjointement avec au moins un objectif.

10. Procédé selon au moins l'une des revendications précédentes, dans lequel, pendant ou après la détection du système cardiovasculaire, une unité de mesure permettant de déterminer le diamètre du système cardiovasculaire est introduite exclusivement par un pore de la coquille calcaire d'un diamètre ≤ 1 mm.

11. Procédé selon au moins l'une des revendications précédentes, dans lequel le sexage est réalisé au moyen de signaux d'absorption et/ou de signaux de fluorescence à travers un pore ≤ 1 mm de l'œuf.

12. Procédé selon au moins l'une des revendications précédentes, dans lequel des signaux complémentaires spécifiques au sexe sont utilisés pour la classification.

13. Procédé selon la revendication 12, dans lequel la classification est mise en œuvre au moyen d'une combinaison/intersection logique des signaux complémentaires spécifiques au sexe par l'intermédiaire de procédés mathématiques, de manière particulièrement avantageuse par l'intermédiaire d'un calcul de quotients.

14. Procédé selon au moins l'une des revendications précédentes, dans lequel le procédé est réalisé entre le troisième et le quatrième jour de couvaison.
